# EUROPÄISCHE PATENTANMELDUNG

(11) **EP 4 529 913 A1**
(43) Veröffentlichungstag der Anmeldung: **02.04.2025**
(21) Anmeldenummer: 24192029.7
(22) Anmeldetag: 31.07.2024
(51) Int. Cl.: A61J 1/20

(54) **SYSTEM ZUR LAGERUNG UND ABGABE EINER MEDIKAMENTENLÖSUNG IN/AUS EINEM MEHRDOSENBEHÄLTER**

(30) Priorität: 27.09.2023 DE 102023126352
(71) Anmelder: Gaplast GmbH, 82442 Altenau (DE)
(72) Erfinder: Kneer, Stephan, 82490 Farchant (DE); Keller, Alexander, 82401 Rottenbuch (DE)
(74) Vertreter: Flosdorff, Jürgen

(57) **Zusammenfassung**

Das System zur Lagerung einer vorzugsweise sterilen Medikamentenlösung in einem Mehrdosenbehälter und zur Abgabe jeweils einer Dosis der Medikamentenlösung an eine Einmalspritze, ist dadurch gekennzeichnet, dass der Mehrdosenbehälter ein Airlessbehälter ist, der aus einem steifen Außenbehälter und einem kontrahierenden Innenbeutel besteht, in den keine Luft zum Druckausgleich bei Abgabe von Medikamentenlösung eintritt, dass an dem Mehrdosenbehälter ein Adapter mit einem Einwegventil und einem weiblichen Luer-Anschluss befestigt ist, und dass die Einmalspritze mit einem passenden männlichen Luer-Anschluss versehen ist, mit dem sie an dem Adapter andockbar ist, um durch Aufziehen der Einmalspritze eine Dosis der Medikamentenlösung aufzunehmen.

## Beschreibung

Die Erfindung betrifft ein System zur Lagerung einer vorzugsweisen sterilen Medikamentenlösung in einem Mehrdosenbehälter und zur Abgabe jeweils einer Dosis der Medikamentenlösung an eine Einmalspritze.

Bisher ist es üblich, eine Medikamentenlösung in einer sogenannten Stechampulle bzw. einem Vial zu lagern, die durch eine Durchstechmembran oder einen Gummipfropfen verschlossen ist, der in der Mitte die geringste Dicke hat. Zur Entnahme einer Dosis der Medikamentenlösung wird der Gummipfropfen oder die Durchstechmembran durch die Nadel einer Standard-Einmalspritze durchstochen, woraufhin durch Aufziehen der Spritze die Dosis der Medikamentenlösung in die Spritze aufgenommen wird. Dabei gibt es einige Schritte, die anfällig für eine Kontamination der Medikamentenlösung und der Spritzennadel sind und das Risiko einer Fehlbedienung in sich bergen. Die Stechampulle kann üblicherweise bis zu 200 ml der Medikamentenlösung aufnehmen, so dass meist zahlreiche Dosen der Medikamentenlösung in eine Einmalspritze aufzunehmen sind und jedes Mal der Stopfen durchstochen wird, so dass Verunreinigungen zu der Medikamentenlösung gelangen können. Außerdem kann die Oberfläche des Stopfens kontaminiert sein. Es kommt zudem vor, dass aus Zeit- und Kostengründen dieselbe Nadel für die Entnahme aus dem Vial und für das Applizieren bei einem Patienten verwendet werden. Bei der Entnahme ist die Stechampulle in Über-Kopf-Lage zu halten, da zum Druckausgleich bei jeder Entnahme Luft in die Ampulle eintritt. Außerdem besteht bei der Entnahme aus der Stechampulle eine nicht unerhebliche Gefahr einer Nadelstichverletzung.

Der vorliegenden Erfindung liegt die Aufgabe zugrunde, ein System anzugeben, bei dem die obigen Nachteile ganz oder größtenteils vermieden sind. Dabei soll sichergestellt sein, dass die Medikamentenlösung vorzugsweise steril in einem Mehrdosenbehälter gelagert werden kann und die einzelnen Dosen in wenigen einfachen Schritten in eine Standard-Einmalspritze aufgenommen werden. Eine Kontamination der Medikamentenlösung soll sowohl innerhalb des Behälters als auch bei der Aufnahme in die Einmalspritze weitestgehend ausgeschlossen sein.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, dass der Mehrdosenbehälter ein Airlessbehälter ist, der aus einem steifen Außenbehälter und einem Innenbeutel besteht, in den keine Luft zum Druckausgleich bei Abgabe von Medikamentenlösung eintritt. Auf dem Mehrdosenbehälter ist ein Adapter mit einem Einwegventil mit einem Luer-Anschluss befestigt, an dem ein Luer-Konus einer Standard-Einmalspritze für ein einfaches und unkompliziertes, sicheres Aufziehen der Spritze mit dem Medikament andocken kann.

Die Ausbildung ist so getroffen, dass der Adapter mit einem weiblichen Luer-Anschluss versehen ist, während die Einmalspritze den passenden männlichen Luer-Anschluss hat, so dass eine dichte Verbindung zwischen dem Mehrdosenbehälter und der Einmalspritze beim Aufziehen der Spritze besteht.

EinTotvolumen innerhalb des Adapters und des Einwegventils kann zudem mit antimikrobiellem Material wie Silber versehen sein, so dass sichergestellt ist, dass keine mikrobielle Verunreinigung zu der Medikamentenlösung in dem Mehrdosen-behälter gelangen kann.

Der bei der Abgabe von Medikamentenlösung an die Einmalspritze entstehende Unterdruck in dem Mehrdosenbehälter wird dadurch ausgeglichen, dass Luft durch eine Öffnung in dem Außenbehälter in den Zwischenraum zwischen dem Außenbehälter und dem sich kontrahierenden Innenbeutel eintritt, so dass keine Luft ins Innere des Innenbeutels gelangt. Damit ist eine vollkommen luftfreie Abfüllung möglich.

Der Mehrdosenbehälter hat einen Behälterhals, auf den der Adapter mit dem Luer-Anschluss und dem Einwegventil vorzugsweise aufgeschnappt, gecrimpt oder geschraubt ist. Das Einwegventil enthält bevorzugt einen etwa glockenförmigen Außenkegel, der bevorzugt aus einem thermoplastischen Elastomer besteht, und die gesamte innere Durchtrittsöffnung des Einwegventils verschließt, bis auf ein Loch, das in der Mitte der Ventilmembran ausgebildet ist, dessen Rand im entspannten Zustand der Ventil membran auf einem Ventilsitzpin aufliegt, der das Loch im entspannten Zustand der Membran dicht verschließt. Hierdurch ist ausgeschlossen, dass Luft von außen ins Innere des Mehrdosenbehälters gelangen kann.

Wenn die Einmalspritze mit ihrem Luer-Anschluss in den konisch sich erweiternden Auslasskanal des Einwegventils eingesetzt ist und die Einmalspritze aufgezogen wird, wird durch den Sog der elastische Außenkegel bzw. die elastische Ventilmembran von dem Ventilsitzpin nach oben abgehoben, wodurch eine Dosis der Medikamentenlösung durch das Loch in die Spritze eingesaugt wird. Wenn an der Innenfläche des weiblichen Luer-Anschlusses zweckmäßigerweise eine antimikrobielle Substanz wie ein Silberring angeordnet ist, ist eine Kontamination der aufgezogenen Medikamentenlösung praktisch ausgeschlossen.

Als weitere Maßnahme zur Sicherstellung einer aseptischen Abfüllung wird mit Vorteil vorgeschlagen, dass die Auslassöffnung des Luer-Adapters durch einen Deckel verschließbar ist, der auf der Oberseite der Auslassöffnung dicht aufliegt, der mit zwei seitlichen Stegen verbunden ist, die an der Außenseite des Adapters angelenkt sind, so dass der Deckel zur Seite schwenkbar ist. Außerdem ist der Deckel mit einem oder zwei biegbaren Federarmen verbunden, die an der Außenseite des Adapters befestigt sind. Der Deckel ist auf der Auslassöffnung seitlich gegen die Kraft der biegbaren Federarme verschiebbar, um die Auslassöffnung für den Luer-Anschluss der Einmalspritze freizugeben, wobei die biegbaren Federarme den Deckel nach dem Entnahmevorgang wieder in seine Verschluss-Stellung verschieben. Der Deckel kann eine Randaussparung aufweisen, in die der Luer-Anschluss der Einwegspritze eingreifen kann, um den Deckel seitlich zu verschieben, was den Vorteil hat, dass der Deckel hierzu nicht mit der Hand des Benutzers berührt werden muss. Auch eine drehbare oder kippbare Version des Deckels ist vorstellbar.

Das erfindungsgemäße System stellt sicher, dass einerseits keine Luft in den Mehrdosenbehälter gelangt, so dass die Medikamentenlösung mit reduziertem oder völlig fehlendem Konservierungsmittelanteil aseptisch abgefüllt werden kann. Das Einwegventil verhindert, dass Luft in den Behälter gelangt, so dass ein Keimwachstum von außen nach innen so gut wie möglich ausgeschlossen ist, wobei Totvolumen in dem Einwegventil durch Silber oder andere antimikrobiellen Materialien kontaminationsfrei gehalten werden kann. Das Aufziehen der Spritze ist wegen der Luer-Verbindung einfach, unkompliziert und sicher, wobei eine Verletzungsgefahr durch die Nadel bei der Aufnahme der Dosis sicher vermieden ist. Beim Einführen der Einmalspritze in den Luer-Adapter des Vials ist zudem keine Überkopf-Lage erforderlich, da sich keine Luft im Inneren des Vials befindet.

Weitere Einzelheiten der Erfindung ergeben sich aus der nachfolgenden Beschreibung bevorzugter Ausführungsformen sowie anhand der Zeichnungen. Dabei zeigen:
- Figur 1A: eine Ausführungsform des erfindungsgemäßen Systems in einer Seitenansicht;
- Figur 1B: die Ausführungsform in einer um 90° gedrehten Darstellung;
- Figur 1C: eine Aufsicht auf die Ausführungsform;
- Figur 1D: einen Vertikalschnitt durch den oberen Bereich der Ausführungsform;
- Figur 2: die Positionen 1 bis 5 bei der Entnahme einer Dosis der Medikamentenlösung;
- Figur 3: einen Vertikalschnitt durch den Adapter und das Einwegventil;
- Figur 4: eine Einzelheit E der Figur 3 im vergrößerten Maßstab;
- Figur 5: eine weitere Ausführungsform des Adapters mit zwei Federelementen in einer Seitenansicht und einer Aufsicht.

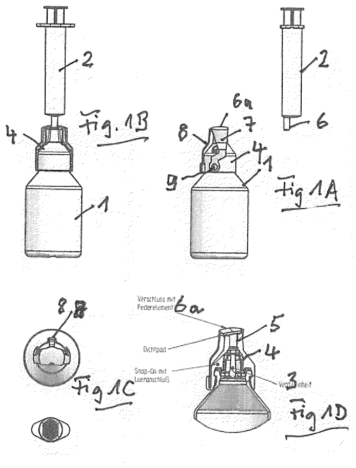

Figur 1A zeigt in einer auseinander gezogenen Darstellung einen Mehrdosenbehälter 1, der eine vorzugsweise sterile Medikamentenlösung enthält, aus dem mit Hilfe einer Einmalspritze 2 jeweils eine Dosis der Medikamentenlösung entnommen wird. Der Mehrdosenbehälter ist ein sogenannter Airlessbehälter, der aus einem steifen Außenbehälter und einem Innenbeutel besteht, der sich nach Entnahme von Medikamentenlösung zusammenzieht, wozu der Außenbehälter mit wenigstens einem Loch/Öffnung versehen ist, durch das jeweils Luft zum Druckausgleich bei Abgabe von Medikamentenlösung eintritt. In den Innenbeutel tritt damit keine Luft zum Druckausgleich ein.

Der Mehrdosenbehälter kann jede geeignete Form haben, beispielsweise eine zylindrische oder bauchige Flaschenform mit einem Behälterhals.

Auf dem Behälterhals des Mehrdosenbehälters 1 ist ein Adapter 4 befestigt, in dem ein Einwegventil 3 angeordnet ist, das weiter unten näher beschrieben wird. Der Auslass des Ventils wird durch einen weiblichen Luer-Anschluss 5 des Adapters 4 gebildet, in den ein männlicher Luer-Anschluss 6 der Einmalspritze 2 dicht eingreifen kann, um eine Dosis der Medikamentenlösung aus dem Mehrdosenbehälter 1 zu entnehmen.

Dabei wird die Einmalspritze 2 zweckmäßigerweise von oben in den Adapter 4 eingeführt, wie Figur 1B zeigt, was deshalb möglich ist, weil in dem Innenbeutel nur die Medikamentenlösung enthalten ist.

Der Luer-Anschluss 5 des Adapters 4 ist im nicht-betätigten Zustand des Systems von einem deckelartigen Verschluss 6a dicht verschlossen, der über beidseitige Stege 7 an der Außenseite des Adapters 4 angelenkt ist und gegen die Kraft einer Feder 8 in eine Öffnungsstellung verschwenkbar ist, um den Luer-Anschluss 5 für die Einmalspritze 2 freizugeben. DerVerschluss 6a ist an seitlichen Lagerzapfen 9 an dem Adapter 4 angelenkt. Die gebogene Feder 8 ist um 90° versetzt an dem Verschluss 6a und an dem Adapter 4 befestigt, und wird bei Öffnung des Verschlusses 6a gespannt, um nach dem Entnahmevorgang den Verschluss 6a wieder in die Schließstellung zurück zu schwenken.

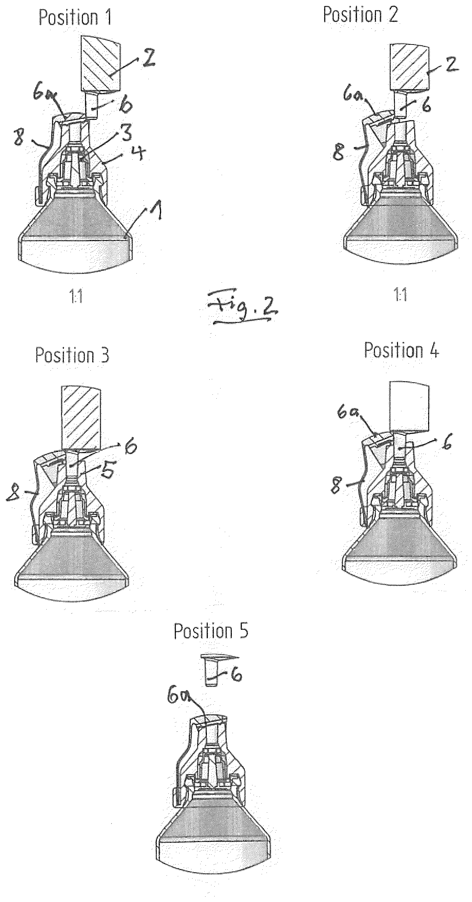

Figur 2 zeigt aufeinanderfolgende Schritte bei der Entnahme von Medikamentenlösung aus dem Mehrdosenbehälter 1. In Position 1 wird die Einmalspritze 2 mit dem männlichen Luer-Anschluss 6 an den Verschluss 6a herangeführt, wozu der Verschluss 6a eine in der Zeichnung nicht dargestellte Rand-Einbuchtung haben kann.

In Position 2 wird der Verschluss 6a von dem Luer-Anschluss 6 gegen die Kraft der Feder 8 zur Seite geschoben, so dass in Position 3 der Luer-Anschluss 6 dicht in den Luer-Anschluss 5 des Adapters 4 eingeführt werden kann. Daraufhin wird die Spritze 2 auf übliche Weise aufgezogen, wobei sie eine Dosis der Medikamentenlösung aus dem Innenbeutel des Mehrdosenbehälters 1 aufnimmt. In Position 5 ist die Einmalspritze 2 entnommen, und der Verschluss 6a ist durch Federkraft wieder in die Schließstellung zurück geschwenkt.

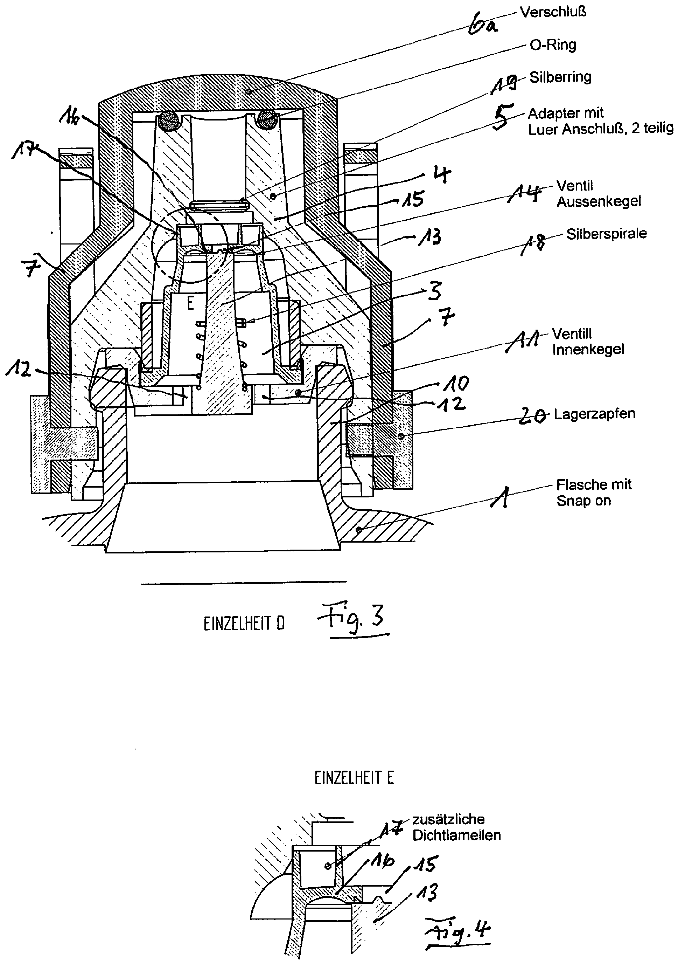

Figur 3 zeigt in vergrößerter Darstellung den Adapter 4 mit Luer-Anschluss 5. Der Adapter 4 ist auf den Hals 10 des Mehrdosenbehälters 1 aufgeschnappt. Die Erfindung ist hierauf aber nicht beschränkt, der Adapter 4 könnte statt dessen auch auf einen Behälterhals aufgeschraubt oder gecrimpt sein.

Der Adapter 4 verjüngt sich kegelstumpfförmig zu dem weiblichen Luer-Anschluss 5, der die Austrittsöffnung des in dem Adapter 4 untergebrachten Einwegventils 3 bildet. Das Einwegventil besteht aus einem Innenkegel 11, der auf die Oberkante des Flaschenhalses 10 aufgesetzt ist und Durchgangsöffnungen 12 für die in dem Mehrdosenbehälter befindliche Medikamentenlösung enthält. Der Ventilinnenkegel enthält außerdem einen mittigen, in Längsrichtung des Adapters sich erstreckenden Ventilsitz-Pin 13, auf dessen Oberseite ein Ventil-Außenkegel 14 aufliegt. Der Ventil-Außenkegel 14 enthält ein mittiges Loch 15, dessen Randbereich im unbetätigten Zustand des Ventils 3 dicht auf der Oberseite des Ventilsitz-Pins 13 aufliegt.

Der Ventil-Außenkegel 14 ist mit seinem unteren Randbereich dicht auf dem Ventil-Innenkegel 11 befestigt und erstreckt sich konisch oder glockenförmig nach oben zulaufend zu seiner oberen Wand mit dem Durchgangsloch 15. Auf der Oberseite der oberen Wand 16 ist eine doppelte Reihe von Dichtlamellen 17 angeordnet, die an der Innenwand des Adapters 4 anliegen.

Im nicht-betätigten Zustand des Systems, d.h. wenn keine Medikamentenlösung entnommen wird, bildet der Ventil-Außenkegel 14 einen dichten Verschluss des Innenraums des Mehrdosenbehälters 1, da die obere Wand 16 des Ventil-Außenkegels 14 dicht auf der Oberseite des Ventilsitz-Pins 13 aufliegt. Wenn jedoch die Einmalspritze 2 mit ihrem männlichen Luer-Anschluss 6 an dem Adapter 4 angedockt ist und die Einmalspritze aufgezogen wird, wird durch die Saugkraft die obere Wand 16 des Ventil-Außenkegels 14 von der Oberseite des Ventilsitz-Pins 13 angehoben, so dass Medikamentenlösung durch die Öffnungen 12 in der Bodenwand des Ventils und durch das obere Loch 15 in dem Ventil-Außenkegel 14 hindurch in die Einmalspritze 2 eingesaugt wird.

Als Sicherungsmaßnahme gegen eine Kontamination der Medikamentenlösung ist eine Silberspirale 18 um den Ventilsitz-Pin 13 gewickelt, und ein Silberring 19 befindet sich im unteren Bereich des weiblichen Luer-Anschlusses 5.

Figur 3 zeigt außerdem, dass der Verschluss 6a des Adapters einstückig mit seinen beiden seitlichen Stegen 7 verbunden ist, die durch Lagerzapfen 20 an dem Gehäuse des Adapters 5 angelenkt sind.

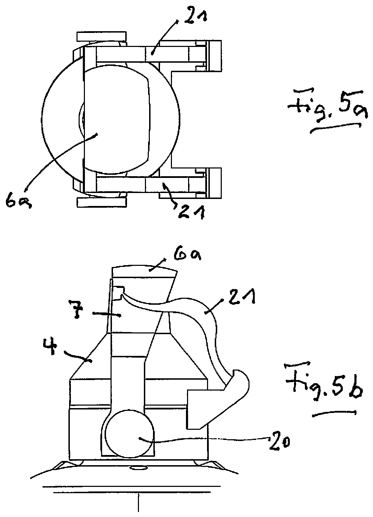

Die Figuren 5A und 5B zeigen bei einer alternativen Ausführungsform, dass zwei geschwungene Federelemente 21 die Stege 7 mit dem Adapter 4 verbinden.

## Patentansprüche

1. System zur Lagerung einer vorzugsweise sterilen Medikamentenlösung in einem Mehrdosenbehälter (1) und zur Abgabe jeweils einer Dosis der Medikamentenlösung an eine Einmalspritze (2),
**dadurch gekennzeichnet,**
**dass** der Mehrdosenbehälter (1) ein Airlessbehälter ist, der aus einem steifen Außenbehälter und einem kontrahierenden Innenbeutel besteht, in den keine Luft zum Druckausgleich bei Abgabe von Medikamentenlösung eintritt,
**dass** an dem Mehrdosenbehälter (1) ein Adapter (4) mit einem Einwegventil (3) und einem weiblichen Luer-Anschluss (5) befestigt ist, und dass die Einmalspritze (2) mit einem passenden männlichen Luer-Anschluss (6) versehen ist, mit dem sie an dem Adapter (4) andockbar ist, um durch Aufziehen der Einmalspritze eine Dosis der Medikamentenlösung aufzunehmen.

2. System nach Anspruch 1,
**dadurch gekennzeichnet,**
**dass** der Mehrdosenbehälter (1) einen Behälterhals hat und dass der Luer-Adapter (4) auf den Behälterhals aufgeschnappt ist.

3. System nach einem der Ansprüche 1 oder 2,
**dadurch gekennzeichnet,**
**dass** der weibliche Luer-Anschluss (5) ein konisch sich erweiternder Auslasskanal des Einwegventils (3) ist.

4. System nach einem der Ansprüche 1 bis 3,
**dadurch gekennzeichnet,**
**dass** das Einwegventil (3) eine elastische Ventilmembran (14) aufweist, die die gesamte innere Durchtrittsöffnung des Einwegventils (3) verschließt bis auf ein Loch (15), mit dessen Rand die Ventilmembran (14) im entspannten Zustand so auf einem Ventilsitz-Pin (13) aufliegt, dass das Einwegventil (3) dicht verschlossen ist.

5. System nach Anspruch 4,
**dadurch gekennzeichnet,**
**dass** die Ventilmembran (14) beim Aufziehen der angedockten Einmalspritze (13) von dem Ventilsitzpin angehoben wird und den Durchtritt von Medikamentenlösung freigibt.

6. System nach einem der Ansprüche 1 bis 5,
**dadurch gekennzeichnet,**
**dass** die Auslassöffnung des Luer-Anschlusses (5) des Adapters (4) durch einen Deckel (6a) verschließbar ist, der mit zwei seitlichen Stegen (7) verbunden ist, die an der Außenseite des Adapters (4) angelenkt sind, wobei der Deckel (6a) außerdem mit wenigstens einem biegbaren Federarm (8, 21) verbunden ist, der beim Verschwenken des Deckels (6a) in die Öffnungsstellung gespannt wird.

7. System nach Anspruch 6,
**dadurch gekennzeichnet,**
**dass** der Deckel (6a) eine Randaussparung aufweist, in die der Luer-Anschluss (6) der Einwegspritze (2) eingreifen kann, um den Deckel (6a) seitlich zu verschieben.

8. System nach einem der Ansprüche 1 bis 7,
**dadurch gekennzeichnet,**
**dass** einTotvolumen innerhalb des Adapters (4) durch ein antimikrobielles Material 18,19) vor Verkeimung geschützt ist.
